# EUROPÄISCHE PATENTSCHRIFT

(11) **EP 2 039 307 B1**
(45) Veröffentlichungstag und Bekanntmachung des Hinweises auf die Patenterteilung: **22.05.2013**
(21) Anmeldenummer: 08164508.7
(22) Anmeldetag: 17.09.2008
(51) Int. Cl.: A61B 17/3205, A61B 17/16

(54) **Medizinisches Instrument zum Ausstanzen von Gewebe**
Medical instrument for stamping out tissue
Instrument médical d'emboutissage de tissus

(30) Priorität: 21.09.2007 DE 102007046397
(43) Veröffentlichungstag der Anmeldung: 25.03.2009
(73) Patentinhaber: Karl Storz GmbH & Co. KG, 78532 Tuttlingen (DE)
(72) Erfinder: Haunschild, Karl-Heinz, 78570 Mühlheim (DE); Schmidberger, Jochen, 72355 Schörzingen (DE)
(74) Vertreter: Witte, Weller & Partner

(56) Entgegenhaltungen:
- WO-A-2007/098354
- DE-A1- 10 342 002
- GB-A- 2 044 103
- US-A- 5 628 762

## Beschreibung

Die Erfindung betrifft ein medizinisches Instrument zum Ausstanzen von Gewebe, mit einem an einem distalen Ende eines Schaftes angeordneten ersten haubenartigen Werkzeug, das proximal eine umlaufende Schneide aufweist, mit einem zweiten Werkzeug, das distal eine umlaufende Schneide aufweist, wobei die beiden Werkzeuge relativ zueinander längs des Schaftes verfahrbar sind, wobei die Schneide des zweiten Werkzeuges in einen inneren Hohlraum des ersten haubenartigen Werkzeuges einfahrbar ist und die beiden Schneiden in Art einer Stanze zusammenwirken.

Ein derartiges medizinisches Instrument ist aus der DE-A-103 42 002 bekannt.

Derartige medizinische Instrumente werden auch als Eierkopf- oder Pilzkopf-Stanzen bezeichnet.

Solche medizinischen Instrumente finden verbreitet Einsatz in der endoskopischen Diagnostik, Chirurgie und Nachsorge der Nasennebenhöhlen und der vorderen Schädelbasis. Sie dienen beispielsweise zur Öffnung der Siebbeinzellen oder der Keilbeinhöhle.

Das erste, am distalen Ende eines Schafts angeordnete Werkzeug ist haubenartig, z.B. pilzkopf- oder eierkopfartig, ausgebildet, so dass es in oder durch kleine Körperöffnungen natürlicher oder künstlicher Art geführt werden kann.

Das zweite Werkzeug weist eine Schneide auf, die der Schneide des ersten Werkzeuges zugewandt ist. Das zweite Werkzeug ist so ausgebildet, dass es zumindest mit seiner Schneide in den inneren Hohlraum des ersten Werkzeuges einfahren kann. Dabei gleiten die umfänglichen Schneiden nahe aneinander vorbei, so dass dazwischen vorhandenes Gewebe getrennt wird. Durch die umlaufenden Schneidekanten arbeiten die beiden Schneiden in Art einer Stanze. Zur Betätigung der Werkzeuge ist meistens eines ortsfest und das andere beweglich, so dass die beiden Werkzeuge zwischen einer offenen Stellung, in der die beiden Schneiden in einem Abstand voneinander liegen, und einem geschlossenen Zustand, in dem die Schneide des zweiten Werkzeuges in die Schneide des ersten Werkzeuges eingefahren ist, bewegt werden können.

Die durch die umlaufenden Schneidekanten festgelegten Ebenen können senkrecht zur Längsachse des Schaftes verlaufen, sie können aber auch geneigt dazu verlaufen.

Der Schaft selbst kann geradlinig oder auch gekrümmt sein, um insbesondere bei Einsatz im HNO-Bereich Zugang zu den Nebenhöhlen finden zu können.

Beim Stanz- oder Schneidevorgang schiebt bzw. zieht das zweite Werkzeug, das in den inneren Hohlraum des ersten Werkzeuges einfährt, das abgetrennte Gewebe in diesen Hohlraum hinein.

Da die Werkzeuge relativ klein sind (übliche Durchmessergrößen eines solchen Stanzkopfes liegen im Bereich von 3 bis 5 mm), ist es sehr schwierig, nach dem Öffnen der Werkzeuge das abgetrennte Gewebe aus dem inneren Hohlraum wieder zu entfernen.

Es ist daher Aufgabe der vorliegenden Erfindung, hier Abhilfe zu schaffen und Maßnahmen vorzusehen, die das Entfernen des Gewebes aus dem inneren Hohlraum des ersten Werkzeuges, also dem Eier- bzw. Pilzkopf, erleichtern.

Erfindungsgemäß wird die Aufgabe dadurch gelöst, dass das zweite Werkzeug distal, und im Abstand zu dessen Schneide, ein plattenförmiges Element trägt, das in etwa den lichten Innenquerschnitt des Hohlraumes des haubenartigen ersten Werkzeuges belegt.

Diese Maßnahme hat den Vorteil, dass das distal vor dem zweiten Werkzeug vorstehende plattenförmige Element vor der Schneide, und noch bevor überhaupt ein Abtrenn- oder Stanzvorgang stattgefunden hat, in den inneren Hohlraum des ersten Werkzeuges hineinläuft. Durch die Ausgestaltung derart, dass es in etwa den lichten Innenquerschnitt belegt, bildet dieses plattenförmige Element quasi eine bewegbare Abschlusswand des inneren Hohlraumes des ersten Werkzeuges.

Beim eigentlichen Schneide- bzw. Stanzvorgang wird Gewebe in den inneren Hohlraum hineinbewegt. Dabei wird auch das plattenförmige Element in den Hohlraum hineinbewegt, allerdings voreilend vor dem Gewebe. Dies geht so weit, bis das plattenförmige Element an ein distales Ende des Hohlraumes stößt.

Beim Öffnen der Werkzeuge wird das im inneren Hohlraum des ersten Werkzeuges aufgenommene Gewebe durch das plattenförmige Element des zweiten Werkzeuges aus dem inneren Hohlraum wieder heraus bewegt.

Das plattenförmige Element wirkt somit als eine Art Räumschild bzw. als eine Abstreif-/Rückhalteplatte. Mit anderen Worten wird das gesamte beim Abtrennvorgang in den inneren Hohlraum des ersten Werkzeuges eingeschobene abgetrennte Gewebe beim Abziehen des zweiten Werkzeuges vom ersten Werkzeug über das plattenförmige Element aus diesem Hohlraum wieder ausgeräumt.

Es ergibt sich somit der Vorteil, dass zwangsläufig beim Öffnen der Werkzeuge das im inneren Hohlraum aufgenommene Gewebe entfernt wird.

Da das plattenförmige Element in einen bodenseitig geschlossenen Hohlraum einfahren muss, belegt es nicht exakt den gesamten lichten Innenquerschnitt, sondern es wird zumindest so viel Raum freigelassen, dass die im Inneren des Hohlraumes vorhandene Luft austreten kann. Dennoch ist sichergestellt, dass bei der entgegengesetzten Bewegung das gesamte Gewebe aus dem inneren Hohlraum ausgeräumt wird.

In einer weiteren Ausgestaltung der Erfindung kommt in einer offenen Stellung der Werkzeuge das plattenförmige Element des zweiten Werkzeuges auf Höhe der proximalseitigen Schneide des ersten Werkzeuges zum Liegen.

Diese Maßnahme hat den Vorteil, dass im geöffneten Zustand der Werkzeuge das plattenförmige Element des zweiten Werkzeuges einen proximalseitigen Abschluss, also quasi einen Abschlussdeckel des inneren Hohlraumes des ersten Werkzeuges, darstellt.

Dies verhindert, dass beispielsweise noch vor dem eigentlichen Stanzvorgang Verunreinigungen in den inneren Hohlraum eindringen können.

In einer weiteren Ausgestaltung der Erfindung kommt in einer geschlossenen Stellung der Werkzeuge das plattenförmige Element des zweiten Werkzeuges auf Höhe eines distalen Endes des Hohlraumes zum Liegen.

Diese Maßnahme hat den Vorteil, dass im geschlossenen Zustand dieses plattenförmige Element einen distalseitigen Abschluss des Hohlraumes bildet.

Mit anderen Worten kann sich das plattenförmige Element über die gesamte axiale Länge des Hohlraumes bewegen, und diesen somit komplett über dessen Länge ausräumen.

In einer weiteren Ausgestaltung der Erfindung weist das plattenförmige Element zumindest eine Aussparung auf.

Diese Maßnahme hat den Vorteil, dass über diese Aussparung gezielt der Druckausgleich beim Hineinbewegen bzw. Herausziehen des plattenförmige Elementes im Hohlraum möglich ist.

In einer weiteren Ausgestaltung der Erfindung ist die zumindest eine Aussparung als Längskerbe am Umfang ausgebildet.

Diese Maßnahme hat den Vorteil, dass ein großflächiges Räumschild zur Verfügung steht, das lediglich eine oder mehrere Längskerben zum Ermöglichen des Luftausgleiches aufweist.

In einer weiteren Ausgestaltung der Erfindung ist das erste haubenartige Werkzeug mit einem stabförmigen Betätigungselement verbunden.

Diese Maßnahme hat den Vorteil, dass über das stabförmige Betätigungselement das erste haubenartige Werkzeug als das bewegliche ausgebildet und hin- und herbewegt werden kann.

In einer weiteren Ausgestaltung der Erfindung ist das zweite Werkzeug an einem Rohrelement des Schaftes angeordnet.

Diese Maßnahme hat den Vorteil, dass das zweite Werkzeug als unbewegliches Werkzeug ausgebildet werden kann, das über den Schaft die Kräfte beim eigentlichen Abtrennvorgang aufnehmen und verteilen kann. Im HNO-Bereich werden ja relativ harte Knorpel- und Knochenstücke entfernt, wozu erhebliche Kräfte notwendig sind.

In einer weiteren Ausgestaltung der Erfindung ist das stabförmige Betätigungselement, das mit dem ersten Werkzeug verbunden ist, in dem Rohrelement, das das zweite Werkzeug trägt, geführt.

Diese Maßnahme hat den Vorteil, dass eine sehr stabile und kompakte Bauweise möglich ist, und dass die beiden Werkzeuge bei den Relativbewegungen exakt geführt werden können, beispielsweise dadurch, dass der Außendurchmesser des stabförmigen Betätigungselements in etwa dem lichten Innendurchmesser des Rohrelementes entspricht.

Dadurch kann auch bei hoher Krafteinwirkung ausgeschlossen werden, dass sich die umlaufenden Schneiden radial versetzen, wodurch das Stanzergebnis negativ beeinflusst würde.

Falls gewünscht ist, dass das erste Werkzeug stationär ist und sich das zweite Werkzeug bewegt, ist das Rohrelement verschieblich und das stabförmige Betätigungselement stationär.

In einer weiteren Ausgestaltung der Erfindung ist das plattenförmige Element am distalen Ende des Rohrelements angebracht.

Diese Maßnahme hat den Vorteil, dass konstruktiv sehr einfach das erfindungsgemäße plattenförmige Element bereitgestellt werden kann, beispielsweise, indem eine entsprechend ausgeformte Ringscheibe an das distale Ende des Rohrelements angeschweißt wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: teilweise geschnitten eine Seitenansicht eines erfindungsgemäßen Instruments;
- Fig. 2: eine etwas vergrößerte Seitenansicht des distalen Endes des Instruments von Fig. 1 mit geschlossenen Werkzeugen;
- Fig. 3: eine der Darstellung von Fig. 2 vergleichbare Darstellung mit geöffneten Werkzeugen;
- Fig. 4: einen Längsschnitt der beiden Werkzeuge in Explosionsdarstellung;
- Fig. 5: einen Schnitt längs der Linie V-V in Fig. 4 in vergrößertem Maßstab;
- Fig. 6: eine perspektivische Ansicht im Wesentlichen von proximal nach distal des ersten Werkzeuges;

- Fig. 7: eine Seitenschnittdarstellung im Bereich des distalen Endes des Instruments von Fig. 1 mit geöffneten Werkzeugen vor Durchführen eines Stanzvorganges an einem Gewebe;
- Fig. 8: eine der Fig. 7 vergleichbare Schnittdarstellung mit geschlossenen Werkzeugen mit ausgestanztem Gewebe; und
- Fig. 9: eine der Darstellung von Fig. 7 vergleichbare Darstellung nach dem Wiederöffnen der Werkzeuge und mit aus dem ersten Werkzeug ausgeräumtem abgetrennten Gewebe.

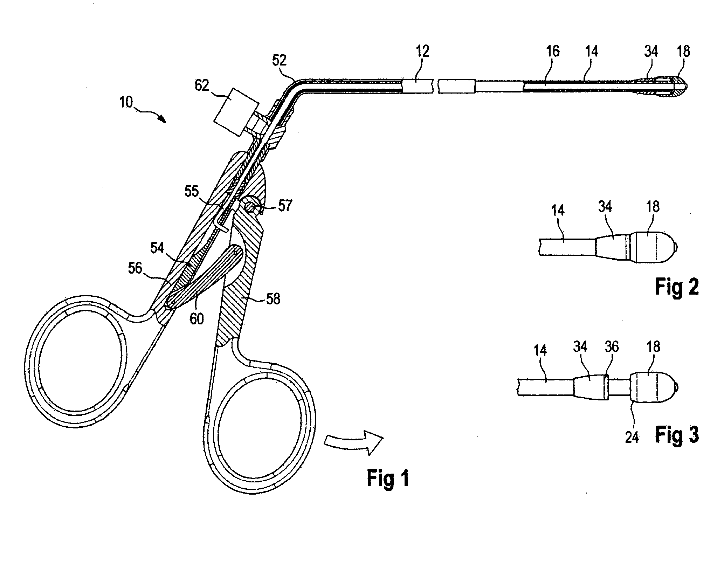

Ein in Fig. 1 dargestelltes erfindungsgemäßes Instrument ist in seiner Gesamtheit mit der Bezugsziffer 10 bezeichnet.

Das Instrument 10 weist einen langerstreckten Schaft 12 auf, der distalseitig ein Rohrelement 14 aufweist.

In dem Inneren des Rohrelementes 14 bzw. dem hohlen Schaft 12 ist ein stabförmiges Betätigungselement 16 aufgenommen.

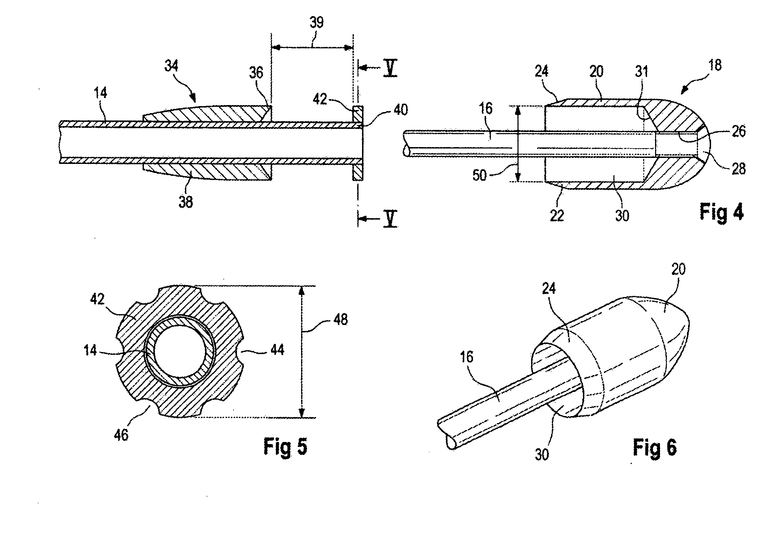

Wie insbesondere aus der Darstellung von Fig. 4 ersichtlich, trägt das stabförmige Betätigungselement 16 distalseitig ein erstes Werkzeug 18.

Das erste Werkzeug 18 weist einen Körper in Form einer Haube 20 auf, die je nach Ausgestaltung eierkopfförmig oder auch pilzkopfförmig sein kann. An ihrem proximalen Ende 22 ist die Haube 20 mit einer umlaufenden Schneide 24 versehen.

Die Haube 20 weist im Bereich ihres Scheitels eine Öffnung 26 auf, in die das stabförmige Betätigungselement 16 eingeschoben ist, bzw. dort über eine Schraube 28 befestigt ist. Diese Verbindung kann auch durch Verschweißen erfolgen.

Somit existiert im Inneren der distalseitig geschlossenen Haube 20 ein Hohlraum 30, dessen distales Ende 31 geschlossen ist, während der Hohlraum 30 proximalseitig offen ist.

Wie ebenfalls aus Fig. 4 zu erkennen, trägt das Rohrelement 14 des Schaftes 12 ein zweites Werkzeug 34. Das zweite Werkzeug 34 trägt distalseitig eine umlaufende Schneide 36.

Das zweite Werkzeug 34 ist als eine Hülse 38 ausgebildet, die auf das Rohrelement 14 aufgeschoben und mit diesem verschweißt ist.

Wie insbesondere aus Fig. 4 zu erkennen ist, steht das Rohrelement 14 distalseitig über das zweite Werkzeug 34 hinaus, und zwar um den Abstand 39.

Am distalen Ende 40 ist ein plattenförmiges Element 42 angebracht, beispielsweise durch Laserschweißen, das die Form einer Ringplatte einnimmt, wie das insbesondere aus der vergrößerten Schnittdarstellung von Fig. 5 ersichtlich ist. Der Außendurchmesser 48 des plattenförmigen Elements 42 entspricht dabei in etwa dem lichten Innendurchmesser 50 des Hohlraumes 30 im ersten Werkzeug 18.

Der Abstand 39, also der Abstand zwischen Schneide 36 des zweiten Werkzeuges 34 und dem plattenförmigen Element 42, entspricht in etwa der Tiefe des Hohlraumes 30.

Wie insbesondere aus Fig. 5 zu entnehmen ist, sind umfänglich an dem plattenförmigen Element 42 diverse Aussparungen 44 vorgesehen, die im dargestellten Ausführungsbeispiel in Form von Längskerben 46 mit kreisabschnittförmigem Querschnitt ausgestaltet sind.

Der Außendurchmesser des stabförmigen Elements 16 entspricht dabei dem lichten Innendurchmesser des Rohrelements 14.

Falls gewünscht ist, dass über das Rohrelement 14 Spülflüssigkeiten zu den Werkzeugen 18, 34 zugeführt werden können, ist der Querschnitt des stabförmigen Elements 16 nicht rund, sondern beispielsweise dreieckig oder längs gekerbt, so dass der Flüssigkeitstransport oder auch ein Gastransport möglich ist.

Aus der Explosionsdarstellung von Fig. 4 ist ersichtlich, dass in dieser Stellung das stabförmige Betätigungselement 16 in das Rohrelement 14 hineingeschoben werden kann.

Hat das plattenförmige Element 42 die Schneide 24 des ersten Werkzeuges 18 erreicht, wobei dies, wie das später noch erläutert werden wird, dem offenen Zustand der Werkzeuge 18 entspricht, stellt das plattenförmige Element 42 einen proximalseitigen Abschluss des Hohlraumes 30 dar. Wird das zweite Werkzeug 36 weiter verschoben, bewegt sich das plattenförmige Element 42 so weit in den Hohlraum 30 hinein, bis dieses auf dessen distales Ende 31 trifft.

Aus den Fig. 4 bis 6 ist auch zu erkennen, dass das etwa ringförmige plattenförmige Element 42 dabei in etwa den lichten Innenquerschnitt 50 des Hohlraumes 30 belegt.

Zurückkehrend zu Fig. 1 ist ersichtlich, dass das stabförmige Betätigungselement 16 über eine Krümmung 52 bis zu einem Schlitten 54 geführt ist. Dort ist dessen abgewinkeltes Ende 55 in den Schlitten 54 eingehängt.

Das stabförmige Betätigungselement 16 ist somit als biegsamer Draht ausgebildet.

Der Schlitten 54 ist in Längsrichtung des stabförmigen Betätigungselements 16 verschieblich in einem ersten Griffteil 56, das das unbewegliche Griffteil darstellt, aufgenommen. Ein zweites Griffteil 58 ist über einen Zapfen 57 gelenkig mit dem ersten Griffteil 56 verbunden.

Zwischen den beiden Griffteilen 56 und 58 erstreckt sich ein Bügel 60, der einerseits mit dem Schlitten 54 im ersten Griffteil 56 und andererseits gelenkig mit dem zweiten beweglichen Griffteil 58 verbunden ist.

Wird das bewegliche Griffteil 58 in die in Fig. 1 dargestellte Stellung bewegt, ist das erste Werkzeug 18 maximal eingezogen, und zwar derart, dass die Schneide 36 des zweiten Werkzeuges 34 an der Schneide 24 des ersten Werkzeuges vorbeigelaufen ist. Dieser Zustand ist in Fig. 1 bzw. in Fig. 2 dargestellt und entspricht der geschlossenen Stellung der beiden Werkzeuge 18, 34.

Wird nun das bewegliche Griffteil 58 in Richtung des in Fig. 1 dargestellten Pfeils bewegt, wird das stabförmige Betätigungselement 16 nach distal verschoben. Somit wird das mit diesem verbundene erste Werkzeug 18 von dem zweiten Werkzeug 34 weg bewegt, wobei dies in Fig. 3 dargestellt ist. Diese Stellung entspricht der offenen Stellung der Werkzeuge 18 und 34.

Wird gewünscht, dass das erste Werkzeug 18 ortsfest ist und sich das zweite Werkzeug 34 bewegt, ist der Schlitten 54 über ein Schubdrahtstück mit dem dann verschiebbar ausgebildeten Rohrelement 14 verbunden. Das Betätigungselement 14 ist dann unverschieblich am Schaft 12 angebracht.

Aus Fig. 1 ist ersichtlich, dass seitlich ein Anschluss 62 vorsteht, über den Spülflüssigkeiten in das Innere des hohlen Schaftes 12 bzw. des rohrförmigen Elementes 14 geführt werden können. Es muss entsprechend dafür Sorge getragen werden, dass diese Flüssigkeiten bis nach distal gelangen können, entweder durch entsprechende Längskerben an der Außenseite des Betätigungselements 16 oder durch die zuvor beschriebene Ausgestaltung mit beispielsweise dreieckförmigem Querschnitt.

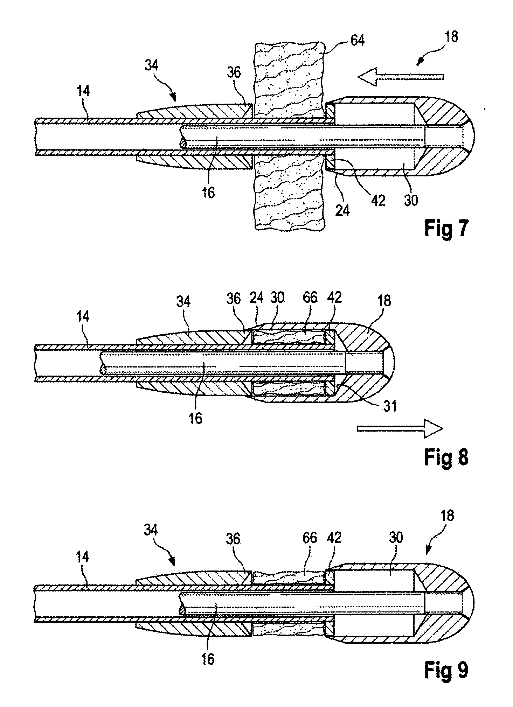

Die Funktionsweise des medizinischen Instruments 10 soll anhand der Bildfolge von Fig. 7 bis 9 näher beschrieben werden. In Fig. 7 sind die beiden Werkzeuge 18 und 34 in geöffnetem Zustand dargestellt, also wie beispielsweise in Fig. 3 beschrieben.

Hier ist ersichtlich, dass das plattenförmige Element 42 einen proximalseitigen Abschluss des inneren Hohlraumes 30 des ersten Werkzeuges 18 bildet, also etwa auf Höhe dessen umlaufender Schneide 24 zum Liegen kommt.

Die Schneide 36 des zweiten Werkzeuges 34 liegt im Abstand, also in dem in Fig. 4 beschriebenen Abstand 39 von der Schneide 24 des ersten Werkzeuges 18. Zwischen den beiden Schneiden 24 und 36 ist nunmehr ein Gewebe 64 vorhanden, das ausgestanzt werden soll.

Die Schneiden 24 und 36 der beiden Werkzeuge 18 und 34 können dabei entweder seitlich an das Gewebe 64 angelegt werden, oder, wenn eine im Gewebe 64 vorhandene kleine Öffnung vergrößert werden soll, kann zunächst das haubenartige erste Werkzeug 18 durch eine solche Öffnung hindurchgestoßen werden. Wird das Betätigungselement 16 nach proximal bewegt, wie das in Fig. 7 durch einen Pfeil angedeutet ist (oder das Werkzeug 34 in umgekehrter Richtung bewegt), bewegt sich die Schneide 24 auf die Schneide 36 zu und trennt somit einen Gewebeabschnitt 66 ab, der, wie das aus Fig. 8 ersichtlich ist, dann im inneren Hohlraum 30 gefangen ist.

Wie ferner aus Fig. 8 zu entnehmen ist, hat sich das plattenförmige Element 42 dabei bis an das distale Ende 31 des Hohlraumes 30 bewegt und bildet somit einen distalen Abschluss des Hohlraumes 30.

Werden nunmehr die Werkzeuge 18 und 34 wieder geöffnet, wie das aus dem Übergang von Fig. 8 zu Fig. 9 ersichtlich ist, schiebt das plattenförmige Element 42 den im Hohlraum 30 aufgenommenen Gewebeabschnitt 66 aus diesem heraus. Somit fungiert das plattenförmige Element 42 quasi als eine Art Räumschild oder Abstreifplatte. In der Position von Fig. 9 kann nunmehr der abgetrennte Gewebeabschnitt 66 problemlos entfernt werden. Ferner ist zu erkennen, dass der Hohlraum 30 völlig leer, d.h. nicht durch Gewebeteile belegt oder verunreinigt ist. Nach Entfernen des Gewebeabschnitts 66 ist das Instrument 10 sofort wieder für einen weiteren Stanzvorgang einsatzfähig.

Die umfänglichen Kerben 46 im plattenförmigen Element 42 (siehe Fig. 5) sorgen dafür, dass die Bewegung unter Luftausgleich stattfinden kann, d.h. die im Hohlraum 30 vorhandene Luft kann über die Kerben 46 aus dem Hohlraum 30 austreten bzw. anschließend wieder eintreten.

Die in den zuvor beschriebenen Ausführungsbeispielen erläuterten Schneiden 24 und 36 verlaufen jeweils in einer Ebene, die senkrecht zur Längsachse des Schaftes 12 bzw. des Rohrelementes 14 verläuft.

Die Ausgestaltung der Erfindung mit dem plattenförmigen Element 42 ist auch bei Schneiden 36 bzw. 24 einsetzbar, die geneigt zu dieser Längsachse verlaufen, wie das beispielsweise bei der eingangs erwähnten DE-A-103 42 002 der Fall ist.

Das plattenförmige Element 42 kann dann, wie im dargestellten Ausführungsbeispiel, sich ebenfalls in einer Ebene erstrecken, die senkrecht zur Längsachse des Rohrelements 14 steht, oder das plattenförmige Element 42 kann entsprechend so geneigt sein, wie es die Schneiden sind.

## Patentansprüche

1. Medizinisches Instrument zum Ausstanzen von Gewebe (64, 66), mit einem an einem distalen Ende (40) eines Schaftes (12) angeordneten ersten haubenartigen Werkzeug (18), das proximal eine umlaufende Schneide (24) aufweist, mit einem zweiten Werkzeug (34), das distal eine umlaufende Schneide (36) aufweist, wobei die beiden Werkzeuge (18, 34) relativ zueinander längs des Schaftes (12) verfahrbar sind, wobei die Schneide (36) des zweiten Werkzeuges (34) in einen inneren Hohlraum (30) des ersten haubenartigen Werkzeuges (18) einfahrbar ist und die beiden Schneiden (24, 36) in Art einer Stanze zusammenwirken, **dadurch gekennzeichnet, dass** das zweite Werkzeug (34) distal und im Abstand (39) zu dessen Schneide (36) ein plattenförmiges Element (42) trägt, das in etwa den lichten Innenquerschnitt (50) des Hohlraumes (30) des haubenartigen ersten Werkzeuges (16) belegt.

2. Medizinisches Instrument nach Anspruch 1, **dadurch gekennzeichnet, dass** in einer offenen Stellung der Werkzeuge (18, 34) das plattenförmige Element (42) des zweiten Werkzeuges (34) auf Höhe der proximalseitigen Schneide (24) des ersten Werkzeuges (18) zum Liegen kommt.

3. Medizinisches Instrument nach Anspruch 1 oder 2, **dadurch gekennzeichnet, dass** in einer geschlossenen Stellung der Werkzeuge (18, 34) das plattenförmige Element (42) des zweiten Werkzeuges (34) auf Höhe eines distalen Endes (31) des Hohlraumes (30) zum Liegen kommt.

4. Medizinisches Instrument nach einem der Ansprüche 1 bis 3, **dadurch gekennzeichnet, dass** das plattenförmige Element (42) zumindest eine Aussparung (44) aufweist.

5. Medizinisches Instrument nach Anspruch 4, **dadurch gekennzeichnet, dass** die zumindest eine Aussparung als Längskerbe (46) am Umfang des plattenförmigen Elements (42) ausgebildet ist.

6. Medizinisches Instrument nach einem der Ansprüche 1 bis 5, **dadurch gekennzeichnet, dass** das erste haubenartige Werkzeug (18) mit einem stabförmigen Betätigungselement (16) verbunden ist.

7. Medizinisches Instrument nach einem der Ansprüche 1 bis 6, **dadurch gekennzeichnet, dass** das zweite Werkzeug (34) an einem Rohrelement (14) des Schaftes (12) angeordnet ist.

8. Medizinisches Instrument nach Anspruch 6 oder 7, **dadurch gekennzeichnet, dass** das stabförmige Betätigungselement (16) im Rohrelement (14) geführt ist.

9. Medizinisches Instrument nach einem der Ansprüche 6 bis 8, **dadurch gekennzeichnet, dass** das plattenförmige Element (42) am distalen Ende des Rohrelements (14) angebracht ist.

## Claims

1. Medical instrument for punching out tissue (64, 66), with a first cap-shaped tool (18) arranged at a distal end (40) of a shaft (12), which proximally has a circular cutting edge (24), with a second tool (34), which distally has a circular cutting edge (36), whereby both tools (18, 34) can be shifted relative to one another along the shaft (12), whereby the cutting edge (36) of the second tool (34) can be introduced into an inner cavity (30) of the first cap-shaped tool (18) and both cutting edges (24, 36) cooperate in the manner of a punch, **characterised in that** the second tool (34) bears a plate-like element (42) distally and at a distance (39) from its cutting edge (36) which approximately covers the clear internal cross-section (50) of the cavity (30) of the cap-shaped first tool (16).

2. Medical instrument of Claim 1, **characterised in that** when the tools (18, 34) are in an open position the plate-like element (42) of the second tool (34) comes to rest at the level of the proximal-side cutting edge (24) of the first tool (18).

3. Medical instrument of Claims 1 or 2, **characterised in that** when the tools (18, 34) are in a closed position the plate-like element (42) of the second tool (34) comes to rest at the level of a distal end (31) of the cavity (30).

4. Medical instrument of anyone of Claims 1 to 3, **characterised in that** the plate-like element (42) has at least one recess (44).

5. Medical instrument of Claim 4, **characterised in that** the at least one recess is designed as a longitudinal notch (46) on the periphery of the plate-like element (42).

6. Medical instrument of anyone of Claims 1 to 5, **characterised in that** the first cap-shaped tool (18) is connected to a rod-like actuation element (16).

7. Medical instrument of anyone of Claims 1 to 6, **characterised in that** the second tool (34) is arranged on a tube element (14) of the shaft (12).

8. Medical instrument of Claims 6 or 7, **characterised in that** the rod-like actuation element (16) is guided within the tube element (14).

9. Medical instrument of anyone of Claims 6 to 8, **characterised in that** the plate-like element (42) is attached at the distal end of the tube element (14).

## Revendications

1. Instrument médical destiné à estamper des tissus (64, 66), comportant un premier outil de type coiffe (18) disposé au niveau d'une extrémité distale (40) d'une tige (12), qui présente côté proximal une lame rotative (24), comportant un second outil (34) qui présente au niveau distal une lame rotative (36), les deux outils (18, 34) pouvant être déplacés l'un par rapport à l'autre le long de la tige (12), la lame (36) du second outil (34) pouvant être introduite dans un espace creux interne (30) du premier outil en forme de cloche (18) et les deux lames (24, 36) collaborant à la façon d'une estampeuse, **caractérisé en ce que** le second outil (34) porte au niveau distal et avec un écart (39) à sa lame (36) un élément en forme de plaque (42) qui occupe approximativement la section interne vide (50) de l'espace creux (30) du premier outil de type coiffe (16).

2. Instrument médical selon la revendication 1, **caractérisé en ce que** dans une position ouverte des outils (18, 34), l'élément en forme de plaque (42) du second outil (34) vient reposer à hauteur de la lame côté proximal (24) du premier outil (18).

3. Instrument médical selon la revendication 1 ou 2, **caractérisé en ce que** dans une position fermée des outils (18, 34), l'élément en forme de plaque (42) du second outil (34) vient à hauteur d'une extrémité distale (31) de l'espace creux (30).

4. Instrument médical selon l'une des revendications 1 à 3, **caractérisé en ce que** l'élément en forme de plaque (42) présente au moins un évidemment (44).

5. Instrument médical selon la revendication 4, **caractérisé en ce que** l'évidement, au moins au nombre de un, est réalisé en tant qu'entaille, encoche ou rainure longitudinale (46) au niveau de la périphérie de l'élément en forme de plaque (42).

6. Instrument médical selon l'une des revendications 1 à 5, **caractérisé en ce que** le premier outil de type coiffe (18) est relié à un élément d'actionnement (16) en forme de tige.

7. Instrument médical selon l'une des revendications 1 à 6, **caractérisé en ce que** le second outil (34) est disposé au niveau d'un élément tubulaire (14) de la tige (12).

8. Instrument médical selon la revendication 6 ou 7, **caractérisé en ce que** l'élément d'actionnement en forme de tige (16) est guidé dans l'élément tubulaire (14).

9. Instrument médical selon l'une des revendications 6 à 8, **caractérisé en ce que** l'élément en forme de plaque (42) est appliqué ou monté au niveau de l'extrémité distale de l'élément tubulaire (14).
